# EUROPEAN PATENT SPECIFICATION

(11) **EP 1 635 820 B1**
(45) Date of publication and mention of the grant of the patent: **21.05.2008**
(21) Application number: 04766041.0
(22) Date of filing: 09.06.2004
(51) Int. Cl.: A61K 31/407, A61P 25/18

(54) **ASENAPINE FOR THE TREATMENT OF SCHIZOPHRENIA IN A PATIENT WITH OVERWEIGHT OR PREDISPOSITION FOR OVERWEIGHT**
ASENAPIN ZUR BEHANDLUNG VON SCHIZOPHRENIE BEI EINEM PATIENTEN MIT ÜBERGEWICHT ODER VERANLAGUNG ZU ÜBERGEWICHT
ASENAPINE DESTINEE AU TRAITEMENT DE LA SCHIZOPHRENIE CHEZ UN PATIENT PRESENTANT UNE SURCHARGE PONDERALE OU UNE PREDISPOSITION A LA SURCHARGE PONDERALE

(30) Priority: 12.06.2003 EP 03101721
(43) Date of publication of application: 22.03.2006
(73) Proprietor: N.V. Organon, 5349 AB Oss (NL)
(72) Inventor: TONNAER, Jeroen A. D. M., N.V. Organon, NL-5340 BH Oss (NL)
(74) Representative: van Wezenbeek, Petrus M.G.F.
(86) International application number: PCT/EP2004/051069
(87) International publication number: WO 2004/110437

(56) References cited:
- WO-A-95/23600
- US-A- 5 763 476
- US-A1- 2002 156 067
- MIGUEL-HIDALGO J J: "ORG-5222 NV ORGANON" CURRENT OPINION IN CPNS INVESTIGATIONAL DRUGS, PHARMA PRESS, LONDON,, GB, vol. 2, no. 1, 2000, pages 85-92, XP009021809 ISSN: 1464-844X
- "ORG-5222; ANTIPSYCHOTIC, DOPAMINE D2 RECEPTOR ANTOGONIST, 5-HT2 RECEPTOR ANTAGONIST" DRUGS OF THE FUTURE, BARCELONA, ES, vol. 18, no. 12, 1993, pages 1117-1123, XP000561700 ISSN: 0377-8282
- SARTORIUS N ET AL: "The usefulness and use of second-generation antipsychotic medications: Preface" CURRENT OPINION IN PSYCHIATRY 2002 UNITED KINGDOM, vol. 15, no. SUPPL. 1, 2002, pages S1-S51, XP008026043 ISSN: 0951-7367
- MCINTYRE R S ET AL: "Antipsychotic metabolic effects: weight gain, diabetes mellitus, and lipid abnormalities." CANADIAN JOURNAL OF PSYCHIATRY. REVUE CANADIENNE DE PSYCHIATRIE. CANADA APR 2001, vol. 46, no. 3, April 2001 (2001-04), pages 273-281, XP008026090 ISSN: 0706-7437
- SUSSMAN N: "Review of atypical antipsychotics and weight gain." THE JOURNAL OF CLINICAL PSYCHIATRY. UNITED STATES 2001, vol. 62 Suppl 23, 2001, pages 5-12, XP008026085 ISSN: 0160-6689
- RUSSELL J M ET AL: "Bodyweight gain associated with atypical antipsychotics: epidemiology and therapeutic implications." CNS DRUGS. NEW ZEALAND 2001, vol. 15, no. 7, 2001, pages 537-551, XP008026091 ISSN: 1172-7047
- BLIN O ET AL: "Antipsychotic-associated weight gain and clinical outcome parameters." THE JOURNAL OF CLINICAL PSYCHIATRY. UNITED STATES 2001, vol. 62 Suppl 7, 2001, pages 11-21, XP008026082 ISSN: 0160-6689
- MALHOTRA SHISHUKA ET AL: "Medical management of obesity associated with mental disorders." THE JOURNAL OF CLINICAL PSYCHIATRY. UNITED STATES 2002, vol. 63 Suppl 4, 2002, pages 24-32, XP008026080 ISSN: 0160-6689
- CASEY D E ET AL: "The pharmacology of weight gain with antipsychotics." THE JOURNAL OF CLINICAL PSYCHIATRY. UNITED STATES 2001, vol. 62 Suppl 7, 2001, pages 4-10, XP008026083 ISSN: 0160-6689
- ALLISON D B ET AL: "ANTIPSYCHOTIC-INDUCED WEIGHT GAIN: A COMPREHENSIVE RESEARCH SYNTHESIS" AMERICAN JOURNAL OF PSYCHIATRY, AMERICAN PSYCHIATRIC ASSOCIATION, WASHINGTON, DC, US, vol. 156, no. 11, November 1999 (1999-11), pages 1686-1696, XP001009543 ISSN: 0002-953X
- BAZIRE S: "Schizophrenia" CHEMIST&DRUGGIST, [Online] 3 August 2002 (2002-08-03), pages 17-20, XP002266562 Retrieved from the Internet: URL:http://www.dotpharmacy.co.uk/up1243.pd f> [retrieved on 2004-01-12]

## Description

The invention relates to the use of asenapine in the manufacture of a medicament for the treatment of schizophrenia administered to a patient with overweight.

The average human population is afflicted with a life-time occurrence of schizophrenia at a rate of about 0.5 - 1 % (Goldber et al.; Canadian J. of Psychiatry Vol 47; pp 833-843; 2002). The disease, if untreated, is completely debilitating with regard to social and economic functioning of the afflicted person. Fortunately, considerable progress has been made during the last 45 years in the treatment of the disease, resulting in some social behaviour benefits for many patients. It is the use of effective anti-psychotic drugs that has produced this dramatic improvement in treatment outcome. With the classic antipsychotic agents, such as chlorpromazine, haloperidol, spiperone etc. the importance of dopamine receptor blockade as the mechanism of action for antipsychotic effects has been demonstrated. These drugs nevertheless had shortcomings which were the challenge to be overcome by new agents. These side effects, collectively referred to as extrapyramidal side (EPS) effects, are Parkinson-like behaviour, akathisia, dystonias and serious, sometimes irreversible disturbances in muscle control, known as tardive dyskinesia (TD). Clozapine, an older drug showed that antipsychotic effects could be obtained without frequent inducement of the afore-mentioned side effects. This led to the class of atypical antipsychotic agents, which are those that are as effective as the first generation of antipsychotic drugs, but less prone to induce extrapyramidal side effects and having a broader therapeutic efficacy. The latter refers to efficacy against the negative symptoms of schizophrenia. The atypical anti-psychotic drugs can be further subdivided into three categories based upon receptor-binding profiles and the side effects that follow. These categories are (a) the relatively pure dopamine antagonists (D2 antagonists, including sulpiride and amisulpiride), (b) the dopamine (D2)-serotonin (5-HT₂)-norepinephrine (alpha 1) antagonists (risperidone, ziprazidone and sertindole) and (c) the multireceptor antagonists (clozapine, olanzapine and seroquel) (See Gerlach and Peacock, International Clinical Psychopharmacology 10 Suppl 3: 39-48, 1995.; Tamminga and Lahti, International Clinical Psychopharmacology 11 Suppl 2: 73-76, 1996). These atypical antipsychotics, however, cause substantial weight gain that is both greater than conventional antipsychotics and of clinically meaningful magnitude. This leads in individual cases to poor compliance and other adverse health effects. The largest weight gains are associated with clozapine and olanzapine, and the smallest with quetiapine and ziprasidone. Risperidone is associated with modest weight changes that are not dose related. Given the equivalent efficacy of atypical antipsychotics, weight-gain profile is a legitimate factor to consider when selecting a treatment (Nasrallah, Psychoneuroendocrinology. Vol 28, Suppl 1 pp 83-96, 2003; Homel, Casey and Allison Schizophrenia Research Vol 55(3) pp 277-284, 2002). This is the more relevant because obesity is commonly seen in patients with schizophrenia. According to some surveys the mean body mass index (BMI) for individuals with schizophrenia is significantly higher than individuals who are not schizophrenic (Homel, Casey and Allison, Schizophrenia Research, Vol 55(3) pp 277-284, 2002; Ananth et al Expert Review of Neurotherapeutics Vol 3(1) pp 59-68, 2003).

US-A-2002/156067 discloses the combined use of a norepinephrine reuptake inhibitor and a neuroleptic agent for the treatment of diseases of the central nervous system.

Sussman (J. Clin. Psychiatry, Vol. 62, Suppl 23, pp. 5-12, 2001) discloses weight-increasing effects of various antipsychotic agents.

Russell and Mackell (CNS drugs. New Zealand, Vol. 15, pp. 537-551, 2001) disclose that atypical antipsychotics are associated with bodyweight gain.

Allison et al (Am. J. Psychiatry, Vol.155, pp.1686-1696, 1999) disclose the effect of a number of antipsychotics on bodyweight.

Bazire (Chemist and Druggist, Vol. 3, pp. 17-20, 2002) discloses weight gain effects for many antipsychotics and that this is considered to be a major problem.

Since overweight and obesity are risk factors for other health diseases, such as diabetes and cardiovascular disorders, the need for an improved drug treatment for individuals having both schizophrenia and overweight is clear.

It has now been found that treatment of such patients with asenapine is safer than treatment with just any one of most other antipsychotic agents.

The term treatment is used here to refer to a measure or set of measures taken and/or prescribed by a doctor in order to combat symptoms or consequences of disease. Treatment with a drug is by administration to the patient by any means known in the art directly to the patient or indirectly by prescription. The benefit of the improved treatment can be observed in individual patients, for example when switching to the new treatment from a treatment with any other antipsychotic agent with weight increasing side effect in that particular patient. The benefit can also be observed as a group effect, whereby it cannot be excluded that certain individuals still gain weight, but the overall group result definitely shows less average weight gain effect in comparison to a known treatment. In view of the favourable property of asenapine on weight it is an aspect of the invention to treat a patient for schizophrenia with asenapine, whereby the patient was having weight gain effect due to another antipsychotic agent. In this aspect of the invention the patient is not necessarily a patient with overweight. In another aspect, the need to avoid weight gain due to drug treatment is related to the special risk effect of overweight in that particular patient, for example due to the presence of other risk factors, for example for diabetes or cardiovascular disease. Such risk factors may stem from genetic disposition or behavioural habits, such as smoking or sudden abstinence from smoking. It is therefore another aspect of the invention to treat a patient for schizophrenia with asenapine, whereby the patient needs to be protected against weight increase due to the presence of risk factors for a disease for which overweight is also a risk factor. Again such a patient in need of avoidance of weight increasing effect is not necessarily already overweight. In still a further aspect the patient is in need of avoidance of weight increasing effect because of the presence of other weight increasing factors per se, such as abstinence from nicotine in relation to a decision to stop the smoking habit.

Schizophrenia is defined in the field of psychiatry as a disease falling in a specific diagnostic category with characteristic cognitive disturbances. Diagnosis can be made in accordance with the criteria given in handbooks for psychiatry, for example in the Diagnostic and Statistical Manual of Mental Disorders 4th edition (DSM-IV) published by the American Psychiatric Association, Washington, D.C. (1994). Antipsychotic agent is a drug with therapeutic activity, be it curative or preventive, on patients with schizophrenia and other psychoses. In this description of the invention no difference is made in meaning between an antipsychotic drug, a neuroleptic drug and an antischizophrenia drug. In the art the terms are used interchangeably, although the term neuroleptic is usually avoided in modern times, since it is associated with the classic drugs having the strongest Parkinson-like side effects, resulting from uncompensated inhibition of dopaminergic neurotransmission in the brain.

Administration to a patient can be by any means aimed at making the drug available near the receptors in the body mediating the therapeutic effect. Tablets and capsules for oral intake are the most commonly known. For asenapine a sublingual formulation is developed so that the drug can be given in the oral cavity and is made available to the general circulation. See for example WO9523600

Obesity and overweight are used in the present context as obesity and overweight according to a judgment by a doctor, so that this term is used here with a medical meaning rather than with a meaning referring to (un)desirable physical appearance of people. Quantitative measures are defined in the art in order to have more objective criteria for overweight and obesity. A commonly used parameter is the body mass index (BMI) defined with the formula G/L²; wherein G is body weight in kg and L is body height in meters. An acceptable BMI from the medical point of view is 25 kg/m². Higher values are considered overweight. Overweight as risk factor for other health problems is proportionally operative as such, that is, the higher the overweight, the higher the chance for emergence of other diseases, such as diabetes and cardiovascular disease. The World Health Organisation and the NIH have defined that the term obesity as a physical condition is characterised by a BMI of ≥ 30 kg/m². Note, for the purpose of clarity of terms that the term overweight includes the term obese, the latter being a more serious form of overweight. Since the effect of overweight is relative to the degree of overweight, different cut-off points than the mentioned 25 kg/m² are used to define unhealthy overweight. One can find in expert literature values of 26 kg/m² and 27.3 kg/m² as starting criteria for obesity in women and 28 and 27.8 as starting criteria for obesity in men. The present invention has as further specific embodiments the use of asenapine for methods of treatment of schizophrenia in individuals with overweight defined objectively as those men selected with a BMI of ≥ 26, ≥ 26.5, ≥ 27, ≥ 27.3, ≥ 27.5, ≥ 28, ≥ 29, ≥ 30, ≥ 35 or ≥ 40 and/or those women selected with a BMI of ≥ 26, ≥ 26.5, ≥ 27, ≥ 27.5, ≥ 27.8, ≥ 28, ≥ 29, ≥ 30, ≥ 35 or ≥ 40.

Asenapine refers to the compound as registered by the WHO under that name, which is chemically named trans-5-chloro-2-methyl-2,3,3a,12b-tetrahydro-1H-dibenz[2,3:6,7]oxepino[4,5-c]pyrrole. It is usually made available as the 1:1 maleate salt, so that asenapine may refer to the maleate salt specifically or to the base, or to any salt or hydrate of the base. In the present description the latter and broad meaning is used. The term encompasses also the separate enantiomeric forms of trans-5-chloro-2-methyl-2,3,3a,12b-tetrahydro-1 H-dibenz[2,3:6,7]oxepino[4,5-c]pyrrole, which can be used in pure form for the same purpose. When amounts of asenapine are given, reference is made to the base content of the substance, unless explicitly stated otherwise.

Asenapine is administered in an amount to the patient in a therapeutically effective amount. By a therapeutically effective amount is meant that amount which is capable of at least partially preventing, reversing, reducing, ameliorating or otherwise suppressing the psychotic disease being treated. The ultimate dosage to provide relief for the patient depends a.o. on individual characteristics, such as condition and age. A therapeutically effective amount can be determined by one of ordinary skill in the art using no more than routine experimentation.

Daily dose is the amount of drug administered per 24 hours in any pharmaceutical formulation. For extended release formulations the intended duration for effective dose administration is divided by the number of days in order to arrive at an indication of the daily dose of the treatment. Asenapine can effectively be used in the category of overweight patients in a daily dose range of 0.5 - 50 mg per person, whereby the exact amount is selected depending on route of administration, desired intensity of effect, and individual patient needs and tolerance. In particular the body weight can influence the daily dose, because portions of the drug may be stored in fat tissue, where it is temporarily not available for the receptors involved in the antischizophrenic effect. The preferred range for daily dose is 5-20 mg. Preferred is to administer the daily dose with a sublingual or buccal formulation in one or more dosage units (see WO 9523600) containing an amount of asenapine selected from the range of from 1- 15 mg, with preference for 5 or 10 mg.

It is an aspect of the invention to provide for the use of asenapine for the manufacture of a medicine, which medicine comprises asenapine and is made to be suitable for the treatment of a patient with overweight, or a patient that was having weight gain effect due to another antipsychotic agent or a patient that needs to be protected against weight increase due to the presence of risk factors for a disease for which overweight is also a risk factor or due to the presence of other weight increasing factors.

### Example

Assessment of the efficacy and safety of asenapine (5 mg twice a day) in subjects with acute exacerbation of their schizophrenic illness, compared to risperidone (3 mg twice daily and placebo in a randomized double blind, fixed-dose 6-week trial. The treatment period of 42 days consisted of a 21-day inpatient phase and a 21-day outpatient phase.

The study was done as a multicenter trial. Patients in the study were recruited in 27 centers and randomly assigned to one of the three trial groups.

Screening of candidate subjects for recruitment into the trial was done immediately after the informed consent was signed. The investigator or subinvestigator determined whether the subject met the DSM-IV criteria for schizophrenia. On day 0, the day before the first dose of trial medication was given, the health status of each subject was re-assessed to ensure eligibility for randomization of the subject in the trial. A total of 182 subjects were included into the trial, of these 180 received treatment: 59 asenapine, 59 risperidone, 62 placebo.

To be considered for inclusion in the trial, male or non-pregnant female subjects of 18 years of age or older and experiencing an acute exacerbation of their schizophrenic illness diagnosed according to DSM IV criteria with schizophrenia of the paranoid type [295.30], disorganised type [295.10], catatonic type [295.20], or undifferentiated type [295.90] were selected. Subjects were to have a PANSS score of at least 60 at baseline.

Asenapine and placebo were prepared as indistinguishable sublingual tablets according to WO9523600, example 1, with adaptation of the amount of asenapine in order to obtain proper amounts into the dosage units.

After a washout period of 3-7 days, subjects in the trial were randomized to the three treatment groups (asenapine, risperidone, placebo). Subjects randomized to the asenapine group received trial medication according to the following schedule: 1 mg twice daily on day 1, 2 mg twice daily on day 2, 3 mg twice daily on day 3, 4 mg twice daily on day 4, and 5 mg twice daily on days 5 through 42. Subjects randomized to the risperidone group received trial medication according to the following schedule: 1 mg twice daily on day 1, 2 mg twice daily on day 2, and 3 mg twice daily on days 3 through 42. Subjects randomized to the placebo group received placebo twice daily throughout the treatment period. Assessments during the treatment period were conducted weekly, except for vital sign assessments during the inpatient phase which were conducted daily.

Among other tests the efficacy on disease symptoms is evaluated with the positive and negative syndrome scale (PANSS). Body weight was measured at screening and on day 42, or the subjects final day of treatment.

Demographic and other subject characterisation were performed and descriptive statistics were obtained for age, weight, and height by treatment group and pooled across treatment groups

**Baseline characteristics by treatment group: age, weight, and height**

| | | Asenapine 5 mg (N=59) | Risperidone 3 mg (N=59) | Placebo (N=62 | Total (N=180) |
|---|---|---|---|---|---|
| Age(years) | N | 59 | 59 | 62 | 180 |
| | Median | 39 | 41 | 41.5 | 41 |
| | Maximum | 70 | 61 | 68 | 70 |
| | Minimum | 21 | 22 | 22 | 21 |
| Weight (kg) | N | 57 | 58 | 58 | 173 |
| | Median | 84.40 | 82.25 | 84.15 | 83.60 |
| | Maximum | 155.0 | 161.9 | 150.0 | 161.9 |
| | Minimum | 58.6 | 57.2 | 54.9 | 54.9 |
| Height (cm) | N | 57 | 57 | 59 | 173 |
| | Median | 173 | 170 | 173 | 172 |
| | Maximum | 188 | 193 | 189 | 193 |
| | Minimum | 152 | 150 | 150 | 150 |
| BMI* | | 28.20 | 28.46 | 28.12 | 28.26 |

| | | | | | |
|---|---|---|---|---|---|
| * BMI was calculated with the median values of weight and height. | | | | | |

### Results

Asenapine and risperidone were both significantly more effective than placebo in reducing the symptoms of schizophrenia. Clinically significant weight gain (i.e., an increase from baseline of ≥7%) was reported in 4% (N=2) of the asenapine subjects, 17% (N=8) of the risperidone-treated subjects, and 2% (N=1) of the placebo-treated subjects.

**Table: Number of subjects with clinically significant change from baseline in body weight by treatment group (All-Subjects-Treated Group)**

| | Asenapine 5 mg | | | Risperidone 3 mg | | | Placebo | | |
|---|---|---|---|---|---|---|---|---|---|
| | N | % | n | N | n | % | N | n | % |
| Increase of ≥ 7% | 46 | 2 | 4.3 | 47 | 8 | 17.0 | 54 | 1 | 1.9 |
| Decrease of ≥ 7% | 46 | 0 | 0.0 | 47 | 1 | 2.1 | 54 | 4 | 7.4 |

| | | | | | | | | | |
|---|---|---|---|---|---|---|---|---|---|
| N is total number of subjects with data and n is number of subjects in category | | | | | | | | | |

Of the 147 subjects in the All-Subjects-Treated Group who had weight data recorded at baseline and at the end of the trial, 11 had a clinically significant increase from baseline in body weight and 5 had a clinically significant decrease from baseline in body weight. The percentage of subjects with clinically significant weight gain from baseline was highest in the risperidone group (17.0%), lower in the asenapine group (4.3%), and lowest in the placebo group (1.9%). Four subjects experienced a greater than 10% weight gain from baseline: 1 asenapine subject, 2 risperidone subjects, and 1 placebo subject..

At end point the risperidone group showed 1.9% increase in body weight from baseline, compared with a 0.5% increase of the asenapine group and a 0.3% increase for the placebo group.

### Overall conclusion

The low incidence of clinically significant weight gain and minimal other side effects demonstrated that asenapine has a favorable benefit-risk profile.

## Claims

1. Use of the antipsychotic agent asenapine in the manufacture of a medicament for the treatment of schizophrenia in a patient wherein the patient is selected from the group of obese patients, patients having a BMI of ≥ 25, patients which were having weight gain due to another antipsychotic agent or patients suffering from diabetes, cardiovascular diseases or abstinence from nicotine.

2. The use of asenapine according to claim 1 wherein the patient is an obese patient.

3. The use of asenapine according to claim 1 wherein the patient has a BMI of ≥ 25.

4. The use of asenapine according to claim 1 wherein the patient was having weight gain due to another antipsychotic agent.

5. The use of asenapine according to claim 1 wherein the patient is suffering from diabetes.

6. The use of asenapine according to claim 1 wherein the patient is suffering from a cardiovascular disease.

7. The use of asenapine according to claim 1 wherein the patient is suffering from abstinence from nicotine.

8. The use of asenapine according to claims 1 or 3 wherein the overweight is defined to be a BMI of ≥ 27.8 for men and ≥ 27.3 for women.

9. The use of asenapine according to claims 1-8 wherein the medicament is to be administered to the patient by the sublingual route.

## Patentansprüche

1. Verwendung des antipsychotischen Agens Asenapin bei der Herstellung eines Medikamentes für die Behandlung von Schizophrenie für einen Patienten, bei dem der Patient aus der Gruppe der fettleibigen Patienten ausgewählt ist; Patienten mit einem BMI ≥ 25, Patienten die einen Gewichtszuwachs aufgrund von anderen antipsychotischen Agenzien haben, oder Patienten, die die an Diabetes, kardiovaskulären Krankheiten oder Abstinenz von Nikotin leiden.

2. Verwendung von Asenapin gemäss Anspruch 1, bei welcher der Patient ein fettleibiger Patient ist.

3. Verwendung von Asenapin gemäss Anspruch 1, bei dem der Patient einen BMI von ≥ 25 hat.

4. Verwendung von Asenapin gemäss Anspruch 1, bei welcher der Patient einen Gewichtszuwachs aufgrund eines anderen antipsychotischen Agents aufweist.

5. Verwendung von Asenapin gemäss Anspruch 1, bei welcher der Patient an Diabetes leidet.

6. Verwendung von Asenapin gemäss Anspruch 1, bei welcher der Patient an einer kardiovaskulären Krankheit leidet.

7. Verwendung von Asenapin gemäss Anspruch 1, bei welcher der Patient an Abstinenz von Nikotin leidet.

8. Verwendung von Asenapin gemäss einem der Ansprüche 1 oder 3, bei dem das Übergewicht definiert ist als ein BMI von ≥ 27.8 für Männer und 27.3 für Frauen.

9. Verwendung von Asenapin gemäss einem der Ansprüche 1-8, bei welcher das Medikament an den Patienten über den sublingualen Weg zu verabreichen ist.

## Revendications

1. Utilisation de l'asénapine comme agent antipsychotique dans la fabrication d'un médicament pour le traitement de la schizophrénie chez un patient, le patient étant sélectionné dans le groupe des patients obèses, des patients ayant un BMI ≥ 25, des patients ayant subi une prise de poids due à l'action d'un autre agent antipsychotique ou des patients souffrant de diabète, de maladies cardiovasculaires ou d'un sevrage à la nicotine.

2. L'utilisation d'asénapine selon la revendication 1, dans laquelle le patient est un patient obèse.

3. L'utilisation d' asénapine selon la revendication 1, dans laquelle le patient a un BMI ≥ 25.

4. L'utilisation d' asénapine selon la revendication 1, dans laquelle le patient a subi une prise de poids due à l'action d'un autre agent antipsychotique.

5. L'utilisation d' asénapine selon la revendication 1, dans laquelle le patient souffre de diabète.

6. L'utilisation d' asénapine selon la revendication 1, dans laquelle le patient souffre d'une maladie cardiovasculaire.

7. L'utilisation d' asénapine selon la revendication 1, dans laquelle le patient souffre d'un sevrage à la nicotine.

8. L'utilisation d'asénapine selon les revendications 1 ou 3, dans laquelle le surpoids est défini comme étant un BMI ≥ 27.8 pour les hommes et ≥ 27.3 pour les femmes.

9. L'utilisation d'asénapine selon les revendications 1 à 8, dans laquelle le médicament est destiné à être administré au patient par voie sublinguale.
